# EUROPÄISCHE PATENTSCHRIFT

(11) **EP 0 714 886 B1**
(45) Veröffentlichungstag und Bekanntmachung des Hinweises auf die Patenterteilung: **07.10.1998**
(21) Anmeldenummer: 95118277.3
(22) Anmeldetag: 21.11.1995
(51) Int. Cl.: C07C 251/54, C07C 249/12, C09D 129/10, C09J 129/10

(54) **Copolymerisierbare Oximether**
Copolymerisable oxime ether
Oxime éther copolymérisable

(30) Priorität: 29.11.1994 DE 4442446
(43) Veröffentlichungstag der Anmeldung: 05.06.1996
(73) Patentinhaber: BASF AKTIENGESELLSCHAFT, 67056 Ludwigshafen (DE)
(72) Erfinder: Karcher, Michael, Dr., D-68723 Schwetzingen (DE); Heider, Marc, Dr., D-67433 Neustadt (DE); Rühl, Thomas, Dr., D-67227 Frankenthal (DE); Henkelmann, Jochem, Dr., D-68165 Mannheim (DE)

(56) Entgegenhaltungen:
- EP-A- 0 617 012
- DE-A- 4 219 385
- J. ORG. CHEM. USSR, Bd. 23, 1987, MOSCOW, Seiten 1285-1288, XP002005847 N.A. NEDOLYA ET AL.: "Vinyl ethers containing an epoxy group. XIV Reaction with oximes"

## Beschreibung

Die vorliegende Erfindung betrifft copolymerisierbare Oximether der allgemeinen Formel I 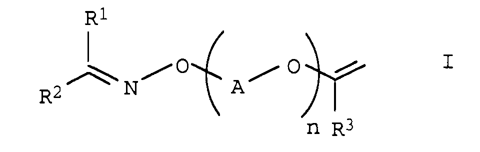 worin die Variablen folgenden Bedeutung haben:
- R¹,R²: unabhängig voneinander Wasserstoff, C₁-C₂₀-Alkyl, C₄-C₁₀-Cycloalkyl, C₆-C₁₀-Aryl oder gemeinsam eine Brücke aus 3 bis 14 Kohlenstoffatomen,
- R³: Wasserstoff, C₁-C₆-Alkyl oder gegebenenfalls inerte Substituenten tragendes Phenyl,
- n: eine ganze Zahl von 1 bis 100,
- A: ein geradkettiger oder verzweigter C₂-C10-Alkylenrest, der Arylgruppen als Substituenten tragen kann.

Weiterhin betrifft die Erfindung ein Verfahren zur Herstellung der Oximether sowie ihre Verwendung in polymeren Beschichtungsmitteln oder Klebstoffen.

Hydroxygruppen enthaltende Oximether sind aus J. Org. Chem. USSR (Engl. Translation), Bd. 23 (1997), Seiten 1285-1288 bekannt.

Polymere Schutzüberzüge oder Klebstoffbeschichtungen mit guten elastischen Eigenschaften, hoher Kohäsion und guter Chemikalien- und Lösemittelbeständigkeit werden vor allem durch vernetzbare Copolymerisate hergestellt. In die Copolymerisate wird dazu durch Copolymerisation ein vernetzungsfähiges Monomer eingebaut.

Diese Monomere sollen demnach eine zur Copolymerisation sowie eine zur Vernetzung geeignete funktionelle Gruppe tragen. Aus der DE-A 42 19 385 sind copolymerisierbare Oximether bekannt, die eine Acrylatgruppe tragen.

Es besteht grundsätzlich ein Bedarf an weiteren vernetzenden Comonomeren, um durch deren Variation die verschiedensten Anforderungen an polymere Beschichtungsmittel und Klebstoffe erfüllen zu können. Weiterhin bestand die Aufgabe, ein Verfahren bereitzustellen, das die Herstellung solcher Verbindungen in technisch einfacher Weise und in hoher Ausbeute erlaubt.

Demgemäß wurden die eingangs definierten Oximether I gefunden. Weiterhin wurde ein Verfahren zu ihrer Herstellung sowie ihre Verwendung als copolymerisierbare Monomere gefunden.

Die Oximether tragen eine vernetzbare Oximgruppe. Die Reste R¹ und R² dieser Gruppe stehen unabhängig voneinander für Wasserstoff oder C₁-C₂₀-Alkyl, wobei C₁-C₆-Alkyl wie Methyl, Ethyl, n-Propyl und n-Butyl bevorzugt ist. Die Alkylgruppen können geradkettig oder verzweigt sein. Weiterhin stehen die Reste R¹ und R² für C₄-C₁₀-Cycloalkyl, wovon Cyclopentyl und Cyclohexyl bevorzugt sind. Auch C₆-C₁₀-Arylgruppen, wie bevorzugt Phenyl, kommen in Betracht, die inerte Substituenten wie Alkyl und Alkoxy tragen können. Die Reste R¹ und R² können gemeinsam eine Brücke mit 3 bis 14 Kohlenstoffatomen bilden, wobei es sich vorzugsweise um Alkylenbrücken handelt. Besonders bevorzugt stehen R¹ und R² jeweils für Methyl, für Methyl und Phenyl oder sie bilden eine C₅-Alkylenbrücke.

Der Rest R³ an der copolymerisierbaren Alkenylgruppe steht bevorzugt für Wasserstoff, es kommen aber auch C₁-C₆-Alkylgruppen wie Methyl und Ethyl sowie Phenyl in Betracht.

Die Variable n gibt die Zahl der Alkylenoxideinheiten in der Oximetherkette an. Diese Zahl kann 1 bis 100 betragen. Bevorzugt sind kurzkettige Verbindungen, in denen n 1 bis 10 beträgt; besonders bevorzugt steht n für 1.

Der zweiwertige Rest A steht für einen geradkettigen oder verzweigten C₂-C₁₀-Alkylenrest, der als Substituenten Arylgruppen tragen kann. Vorzugsweise steht A für eine Ethylengruppe.

Die erfindungsgemäßen Oximether der Formel I werden aus Oximetheralkoholen der Formel II 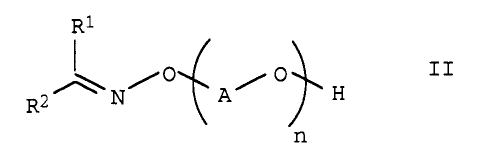 in der die Variablen die oben angegebene Bedeutung haben, hergestellt. Diese Oximetheralkohole sind bekannt oder nach bekannten Methoden herstellbar (z.B. Bachman et al., J. Am. Chem. Soc. 81 (1959) 4223). Als Ausgangsverbindungen zu ihrer Herstellung dienen Oxime, die aus Ketonen bzw. Aldehyden und Hydroxylamin hergewerden können (Houben-Weyl, Methoden der Org. Chemie, Thieme Verlag, 1968, Bd. 10/4, S. 10ff).

Die Oxime können mit Alkylenoxiden wie Ethylenoxid, Propylenoxid und Butylenoxid, aber auch mit Styroloxid in Gegenwart einer Base wie Lithiumethylat umgesetzt werden. Um n Alkylenoxideinheiten in den Oximetheralkoholen der Formel II zu erhalten, werden n Äquivalente des entsprechenden Epoxids mit einem Äquivalent Oxim umgesetzt.

Die Oximetheralkohole der Formel II werden mit einem Alkin der Formel III umgesetzt.

R³ ― C ≡ CH III

Vorzugsweise handelt es sich bei diesem Alkin um Acetylen, aber auch Alkine wie Propin oder Phenylacetylen kommen in Betracht. Wenn Acetylen eingesetzt wird, kann dieses unverdünnt oder mit inerten Gasen wie Stickstoff, Methan oder Argon verdünnt verwendet werden.

Das molare Verhältnis des Oximetheralkohols II zum Alkin III beträgt im allgemeinen 0,05:1 bis 1,2:1, bevorzugt 0,2:1 bis 0,8:1.

Die Reaktion wird in Gegenwart einer anorganischen Base durchgeführt. Dazu kommen Alkalimetall- und Erdalkalihydroxide wie Lithiumhydroxid, Natriumhydroxid und Calciumhydroxid, bevorzugt aber Kaliumhydroxid in Betracht. Weiterhin können Alkalimetallalkoholate, vorzugsweise C₁-C₄-Alkoholate wie Natriummethylat, Natriumethylat, Kaliummethylat und Kalium-tert.-butylat verwendet werden. In der Regel werden 0,005 bis 1 mol Base, vorzugsweise 0,02 bis 0,3 mol pro Mol Oximetheralkohol II eingesetzt.

Obwohl die Umsetzung ohne Lösungsmittel ausgeführt werden kann, hat es sich als vorteilhaft erwiesen, in einem Lösungsmittel zu arbeiten, wobei polare aprotische Lösungsmittel wie Dimethylsulfoxid, Phenylmethylsulfoxid, Sulfolan und Polyethylenglykole sowie deren Ether besonders bevorzugt werden. Die Menge des Lösungsmittels kann in weiten Grenzen variiert werden und kann 10 bis 90 Gew.-% des Reaktionsgemisches ausmachen.

Die Reaktionstemperatur kann 50 bis 250°C, bevorzugt 70 bis 120°C betragen. Der Reaktionsdruck kann 1 bis 100 bar betragen.

Die Oximetheralkohole, die Base und das Lösungsmittel können vermischt werden und bei Reaktionstemperatur mit dem Alkin versetzt werden. Die Reaktion ist im allgemeinen nach 1 bis 36 h beendet. Die Isolierung der Verfahrensprodukte kann bei relativ flüchtigen Verbindungen destillativ erfolgen, längerkettige Verbindungen der Formel I können auch ohne weitere Reinigung verwendet werden. Die im Destillationssumpf befindliche Base kann in die Reaktion zurückgeführt werden.

Die Verfahrensprodukte der Formel I finden Verwendung als copolymerisierbare Monomere für die Herstellung von polymeren Beschichtungsmitteln und Klebstoffen. Sie können dazu in an sich bekannter Weise in Mengen von vorzugsweise 0,01 bis 30 Gew.-% bezogen auf die gesamten Monomeren radikalisch oder kationisch polymerisiert werden, insbesondere mit C₁-C₁₀-Alkyl(meth)acrylaten, Vinylestern von 1 bis 20 C-Atome enthaltenden Carbonsäuren und Vinylethern von C₁-C₂₀-Alkoholen wie Methyl-, Ethyl-, Isobutyl- und Octadecylvinylether.

### Beispiele

### Beispiel 1

### Herstellung von 2-(Vinyloxy)-ethylacetonoxim

In einem 300 ml Autoklav, der 75 g (0,64 mol) Hydroxyethylacetonoxim, 9,4 g (0,17 mol) KOH und 75 g Sulfolan enthielt, wurde ein Stickstoffdruck von 5 bar angelegt. Bei 90°C wurde Acetylen bis zu einem Gesamtdruck von 20 bar aufgepreßt. Verbrauchtes Acetylen wurde durch Nachpressen ersetzt. Nach 24 h wurde entspannt und der Reaktionsaustrag wurde destilliert. Es wurden 88,9 g 2-(Vinyloxy)-ethylacetonoxim (97 % Ausbeute) mit einem Siedepunkt von 45°C (7,5 mbar) isoliert.

### Beispiel 2

### Herstellung von 2-(Vinyloxy)-ethylacetonoxim

Analog zu Beispiel 1 wurden 30 g (0,26 mol) Hydroxyethylacetonoxim, 3,8 g (0,07 mol) KOH und 120 g Dimethylsulfoxid bei 90°C umgesetzt. Die Ausbeute betrug 84 %.

### Beispiel 3

### Herstellung von 2-(Vinyloxy)-propylacetophenonoxim

Analog zu Beispiel 1 wurden 30 g (0,16 mol) Hydroxypropylacetophenonoxim, 3,8 g (0,07 mol) KOH und 120 g Dimethylsulfoxid bei 90°C umgesetzt. 2-(Vinyloxy)-propylacetophenonoxim wurde in einer Ausbeute von 78 % mit einem Siedepunkt von 110°C (5 mbar) isoliert.

### Beispiel 4

### Polymerisation von 2-(Vinyloxy-)ethylacetonoxim mit Maleinsäureanhydrid

275 g Toluol, 24 g Maleinsäureanhydrid und 35 g 2-(Vinyloxy)-ethylacetonoxim wurden bis zum Rückfluß erhitzt, innerhalb von 2 Stunden eine Lösung von 0,59 g tert.-Butylperoktoat in 20 g Toluol zugetropft und 1 Stunde nachgerührt. Anschließend wurde abgekühlt, das Polymerisat mit Cyclohexan gefällt, abgesaugt und bei 50°C im Vakuum getrocknet.

Ausbeute: 32 g (55 %) (bräunliches Pulver).

## Patentansprüche

1. Oximether der allgemeinen Formel I 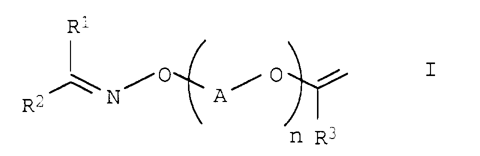 worin die Variablen folgenden Bedeutung haben:
R¹,R² unabhängig voneinander Wasserstoff, C₁-C₂₀-Alkyl, C₄-C₁₀-Cycloalkyl, C₆-C₁₀-Aryl oder gemeinsam eine Brücke aus 3 bis 14 Kohlenstoffatomen,
R³ Wasserstoff, C₁-C₆-Alkyl oder gegebenenfalls inerte Substituenten tragendes Phenyl,
n eine ganze Zahl von 1 bis 100,
A ein geradkettiger oder verzweigter C₂-C₁₀-Alkylenrest, der Arylgruppen als Substituenten tragen kann.

2. Oximether nach Anspruch 1, in denen die Reste R¹ und R² jeweils für Methyl stehen, für Methyl und Phenyl stehen, oder R¹ und R² eine Brücke aus 5 Kohlenstoffatomen bilden.

3. Oximether nach Anspruch 1 oder 2, in denen der Rest R³ für Wasserstoff steht.

4. Oximether nach den Ansprüchen 1 bis 3, in denen die Variable n 1 ist.

5. Verfahren zur Herstellung von Oximethern der allgemeinen Formel I gemäß Anspruch 1, dadurch gekennzeichnet, daß man Oximetheralkohole der allgemeinen Formel II 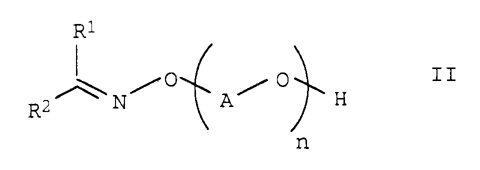 in denen die Variablen die oben angegebene Bedeutung haben, mit einem Alkin der allgemeinen Formel III
R³ ― C ≡ CH III
in der R³ die oben angegebene Bedeutung hat, in Gegenwart einer anorganischen Base umsetzt.

6. Verfahren nach Anspruch 5, dadurch gekennzeichnet, daß man die Umsetzung in Dimethylsulfoxid oder Sulfolan durchführt.

7. Verfahren nach Anspruch 4 oder 5, dadurch gekennzeichnet daß man als Base Kaliumhydroxid oder Alkalimetallalkoholate verwendet.

8. Verwendung von Oximethern der Formel I gemäß Anspruch 1 als Comonomer für die Herstellung von polymeren Beschichtungsmitteln oder Klebstoffen.

## Claims

1. An oxime ether of the general formula I 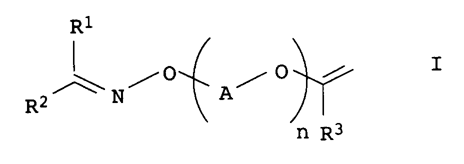 where the variables have the following meanings:
R¹,R² independently of one another hydrogen, C₁-C₂₀-alkyl, C₄-C₁₀-cycloalkyl, C₆-C₁₀-aryl or together a bridge of 3 - 14 carbon atoms,
R³ hydrogen, C₁-C₆-alkyl or phenyl which is unsubstituted or carries inert substituents,
n an integer from 1 to 100,
A a straight-chain or branched C₂-C₁₀-alkylene radical which can carry aryl groups as substituents.

2. An oxime ether as claimed in claim 1, where R¹ and R² are each methyl, or are methyl and phenyl, or R¹ and R² form a bridge of 5 carbon atoms.

3. An oxime ether as claimed in claim 1 or 2, where R³ is hydrogen.

4. An oxime ether as claimed in any of claims 1 to 3, where n is 1.

5. A process for preparing oxime ethers of the general formula I as claimed in claim 1, which comprises reacting oxime ether alcohols of the general formula II 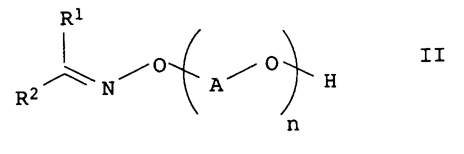 where the variables have the abovementioned meanings, with an alkyne of the general formula III
R³ ― C ≡ CH III
where R³ has the abovementioned meaning, in the presence of an inorganic base.

6. A process as claimed in claim 5, wherein the reaction is carried out in dimethyl sulfoxide or sulfolane.

7. A process as claimed in claim 4 or 5, wherein potassium hydroxide or alkali metal alcoholates are used as base.

8. The use of oxime ethers of the formula I as claimed in claim 1 as comonomers for preparing polymeric coating compositions or adhesives.

## Revendications

1. Ethers d'oximes de formule générale I 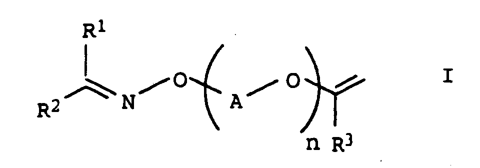 dans laquelle les variables ont les significations suivantes :
R¹ et R², indépendamment l'un de l'autre, sont des atomes d'hydrogène, des groupes alkyle en C₁-C₂₀, cycloalkyle en C₄-C₁₀, aryle en C₆-C₁₀, ou forment ensemble un pont constitué de 3 à 14 atomes de carbone,
R³ est un atome d'hydrogène, un groupe alkyle en C₁-C₆ ou un groupe phényle portant éventuellement des substituants inertes,
n est un nombre entier de 1 à 100,
A est un radical alkylène en C₂-C₁₀ à chaîne droite ou ramifiée, qui peut porter des groupes aryle en tant que substituants.

2. Ethers d'oximes selon la revendication 1, dans lesquels les radicaux R¹ et R² représentent chacun le groupe méthyle, le groupe méthyle et le groupe phényle, ou encore R¹ et R² forment un pont constitué de 5 atomes de carbone.

3. Ethers d'oximes selon la revendication 1 ou 2, dans lesquels le radical R³ est un atome d'hydrogène.

4. Ethers d'oximes selon l'une quelconque des revendications 1 à 3, dans lesquels la variable n vaut 1.

5. Procédé de préparation d'éthers d'oximes de formule générale I selon la revendication 1, caractérisé en ce qu'on fait réagir, en présence d'une base minérale, des éthers alcooliques d'oximes de formule générale II 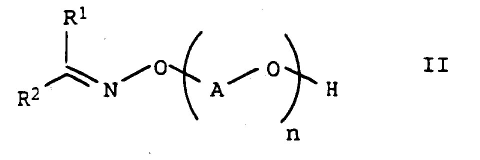 où les variables ont les significations données ci-dessus, avec un alcyne de formule générale III
R³ - C ≡ CH III
dans laquelle R³ a les significations données ci-dessus.

6. Procédé selon la revendication 5, caractérisé en ce qu'on met en oeuvre la réaction dans du diméthylsulfoxyde ou du sulfolanne.

7. Procédé selon la revendication 4 ou 5, caractérisé en ce qu'on utilise en tant que base de l'hydroxyde de potassium ou un alcoolate d'un métal alcalin.

8. Utilisation d'éthers d'oximes de formule I selon la revendication 1 en tant que comonomère pour préparer des agents de revêtement ou des adhésifs polymères.
